# EUROPEAN PATENT SPECIFICATION

(11) **EP 0 473 088 B1**
(45) Date of publication and mention of the grant of the patent: **21.01.1998**
(21) Application number: 91114255.2
(22) Date of filing: 26.08.1991
(51) Int. Cl.: C08F 283/00, C08G 73/02

(54) **Non-crosslinked, polybranched polymers**
Nichtvernetzte, vielfach verzweigte Polymere
Polymères non-réticulés, multiplement ramifiés

(30) Priority: 27.08.1990 US 573362; 01.08.1991 US 739167
(43) Date of publication of application: 04.03.1992
(73) Proprietor: DENDRITECH INCORPORATED, Midland, MI 48642 (US)
(72) Inventor: Hedstrand, David M., Midland, Michigan 48640 (US); Tomalia, Donald A., Midland, Michigan 48640 (US)
(74) Representative: Spott, Gottfried, Dr.

(56) References cited:
- EP-A- 0 234 408
- WO-A-88/01180
- US-A- 4 857 599
- DATABASE WPI Week 7724, Derwent Publications Ltd., London, GB; & JP-A-52 054 800 (RESEARCH INST PRODN)

## Description

### BACKGROUND OF THE INVENTION

This invention deals with non-crosslinked, poly-branched polymers having a comb-burst configuration and a process for preparing such polymers.

Macromolecular organic compounds having novel structures have been investigated for many years as academic curiosities and very little attention has been paid to their use in industrial applications. Since the early 1980's, there has been a renewed interest in the study and development of such macromolecular materials in order to control their critical molecular design parameters, for example, size, shape, surface chemistry, flexibility and topology, for use in industrial applications. These materials have found such diverse uses as demulsifiers for oil-in-water emulsions, as wet strength agents in the manufacture of paper, as agents for modifying viscosities in aqueous formulations such as paints and as submicron size calibrators. Certain biological uses have also been suggested for these materials.

Structurally, polymers are classified as either linear or branched wherein the term "branched" generally means that the individual molecular units of the branches are discrete from the polymer backbone, yet may have the same chemical constitution as the polymer backbone. Thus, regularly repeating side groups which are inherent in the monomeric structure and are of different chemical consitutution than the polymer backbone are not considered as "branches", that is, for example, the methyl groups pendent on a polydimethylsiloxane chain are not considered to be branches of that polymer.

In U.S. Patent 4,507,466, issued March 26, 1985, the patentees therein described the prepartion of polymers having "branching" in the following manner:

"To produce a branched polymer, it is necessary to employ an initiator, a monomer, or both that possess at least three moieties that function in the polymerization reaction. Such monomer or initiators are often called polyfunctional. The simplest branched polymers are the chain branched polymers wherein a linear backbone bears one or more essentially linear pendant groups. This simple form of branching, often called comb branching, may be regular wherein the branches are uniformly and regularly distributed on the polymer backbone or irregular wherein the branches are distributed in non-uniform or random fashion on the polymer backbone." "An example of regular comb branching is a comb branched polystyrene as described by T. Altores et al. in J. Polymer Sci., Part A, Vol. 3 4131-4151 (1965) and an example of irregular comb branching is illustrated by graft copolymers as described by Sorenson et al. in "Preparative Methods of Polymer Chemistry", 2nd Ed., Interscience Publishers, 213-214 (1968).

Another type of branching is exemplified by crosslinked or network polymers wherein the polymer chains are connected via tetravalent compounds, e.g., polystyrene molecules bridged or cross-linked with divinylbenzene. In this type of branching, many of the individual branches are not linear in that each branch may itself contain groups pendant from a linear chain. More importantly in network branching, each polymer macromolecule (backbone) is cross-linked at two or more sites to other polymer macromolecules. Also the chemical constitution of the cross-linkages may vary from that of the polymer macromolecules. In this so-called cross-linked or network branched polymer, the various branches or cross-linkages may be structurally similar (called regular cross-linked) or they may be structurally dissimilar (called irregularly cross-linked). An example of regular cross-linked polymers is a ladder-type poly(phenylsilsesquinone) [sic] {poly(phenylsilsesquioxane)}."

Sogah, et al., in the background of U.S. Patent 4,544,724, issued October 1, 1985, discusses some of these types of polymers and gives a short review of the many publications and disclosures regarding them.

One of the inventors herein, Donald A. Tomalia, and many of his co-workers have been working in this field for several years and have issued many patents which disclose various non-crosslinked, macromolecular branched assemblies.

For example, U.S. Patent 4,435,548, issued March 6, 1984 discusses branched polyamidoamines; U.S. Patent 4,507,466, issued March 26, 1985, U.S. Patent 4,558,120, issued December 10, 1985, U.S. Patent 4,568,737, issued February 4, 1986, U.S. Patent 4,587,329, issued May 6, 1986, U.S. 4,713,975, issued December 22, 1987, U.S. Patent 4,871,779, issued October 3, 1989, and U.S. Patent 4,631,337, issued December 23, 1986, discuss the preparation and use of dense star polymers, and U.S. Patent 4,737,550, issued April 12, 1988 and U.S. Patent 4,857,599, issued August 15, 1989, discuss bridged and other modified dense star polymers.

Also, other structural configurations of macromolecular materials that have been disclosed include star/comb-branched polymers, such disclosure being found in U.S. Patents 4,599,400, issued July 8, 1986 and U.S. Patent 4,690,985, issued September 1, 1987, and finally, rod-shaped dendrimer polymers are disclosed in U.S. Patent 4,694,064, issued September 15, 1987.

The polyamidoamines referred to supra are also disclosed in U.S. Patent 4,758,635, issued July 19, 1988 to Wilson et al.

Hutchins, et al, in U.S. Patents 4,847,328, issued July 11, 1989 and U.S. Patent 4,851,477, issued July 25, 1989, deal with hybrid acrylic-condensation star polymers and Joseph et al in U.S. Patents 4,857,615, issued August 15, 1989, U.S. Patent 4,857,618, issued August 15, 1989, and U.S. Patent 4,906,691, issued March 6, 1990, deal with condensed phase polymers which are linear polymers having regularly, or irregularly, spaced polymeric branches, essentially on the order of a comb structure macromolecule.

An excellent presentation of the structures and chemistries of many such macromolecular branched assemblies can be found in Tomalia, D.A., Naylor, A.M., and Goddard, W.A. III, Angewandte Chemie, 29/2 (1990), pages 138 to 175.

However, none of the disclosures of the prior art deal with the novel polymers of the instant invention which are non-crosslinked, poly-branched polymers. For simplicity sake, the polymers of the instant invention can be generally characterized as multiple polymeric branches on multiple polymeric branches.

### BRIEF DESCRIPTION OF THE DRAWINGS

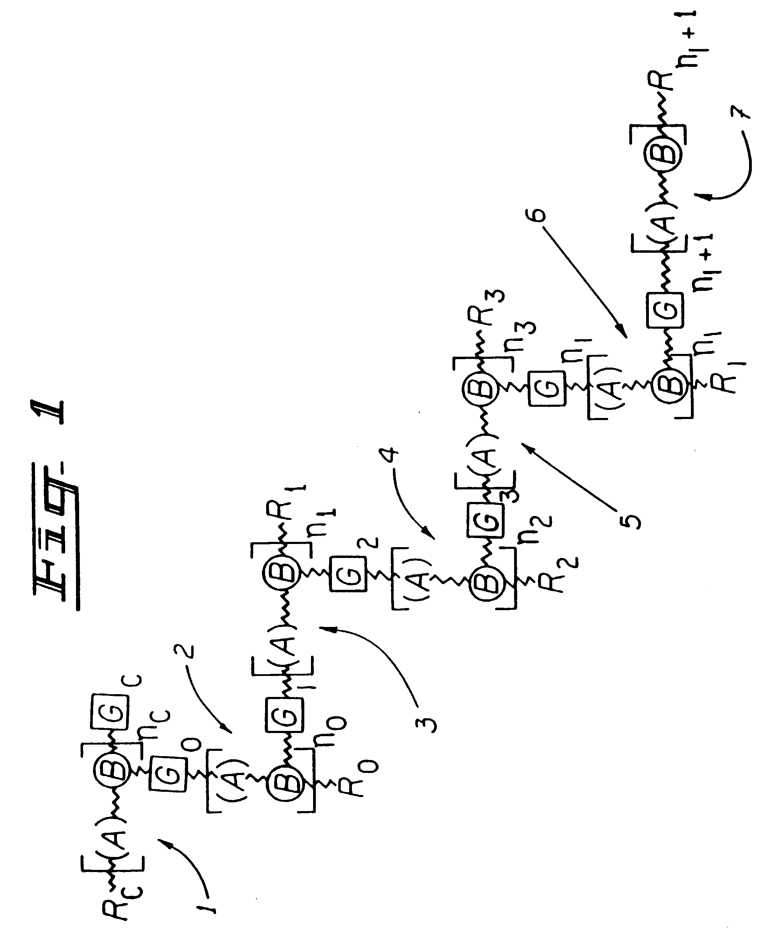

Fig. 1 is a schematic in two dimensions of the polymer configuration of the polymers of the instant invention wherein
1 is the initiator core (initiator core molecule);
2 is first grafting and first branching and generation 0;
3 is second grafting and second branching and generation 1;
4 is third grafting and third branching and generation 2;
5 is fourth grafting and fourth branching and generation 3;
6 is (i + 1)^{th} grafting and (i + 1)^{th} branching and generation i, and
7 is (i + 2)^{th} and all iterative grafting and (i + 2)^{th} and all interative branching, and generation (i + 1) and all subsequent generations.

### THE INVENTION

Benefits and other perceived advantages are achieved in accord with the present invention which comprises novel non-crosslinked, poly-branched polymers, and methods for manufacturing such polymers. In its broadest scope, this invention deals with poly-branched polymers having at least one branch referred to herein as a "core branch" emanating from a core molecule, said branch being essentially linear, and having at least one end chemically coupled to the core molecule, with the other end of the branch terminating in a group from a molecule used to initiate the reaction by which the branch was prepared, and at least one second branch which is branched from the core branch, said second branch, or branches, being essentially linear, and having at least one end chemically coupled to the core branch, with the other end of the branch terminating in a group selected from a molecule used to prepare the second branch polymer, which when subjected to iterative polymer grafting steps (i.e. generations, which will be delineated further herein), form three-dimensional organizations of ordered organic molecules. These polymers are hereinafter referred to as "comb-burst" structures in that they are prepared from comb-like core molecules, but after subsequent grafting of additional branches pursuant to the processes of this invention, tend to have the appearance in two dimensions of a woven wire fence, which when viewed in three dimensions gives a topology having a starburst-like appearance. Hence, "comb-burst".

This invention therefore comprises a composition of matter comprising non-crosslinked poly-branched polymers having the general formula 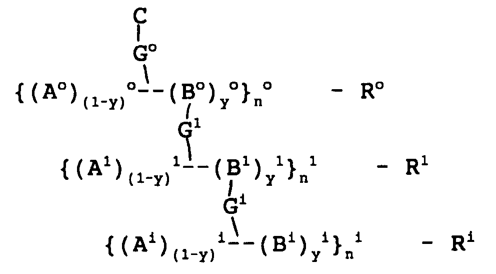 wherein:
C is a core molecule;
each R is the residual moiety of an initiator selected from a group consisting of free radical initiators, cationic initiators, anionic initiators, Lewis acid and group transfer initiators;
A and B are polymerizable monomers or comonomers capable of withstanding the conditions required for preparation of a graft polymer or for preparation of subsequent graft junctures;
each G is a grafting component, and the designation 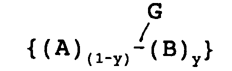 indicates that G can attach to either an (A) unit or a (B) unit;
n is the degree of polymerization of the indicated generation comb branches,
y is the fraction of B units in the indicated generation branch, and has a value of .01 to 1;
the superscripts 0, 1 and i designate the comb branch generation level, the superscripts 0, 1 and i designate the comb burst generation level;
and at least n° and n¹ are > 2.

In a preferred embodiment, the invention comprises a composition of matter comprising non-crosslinked poly-branched polymers having the general formula 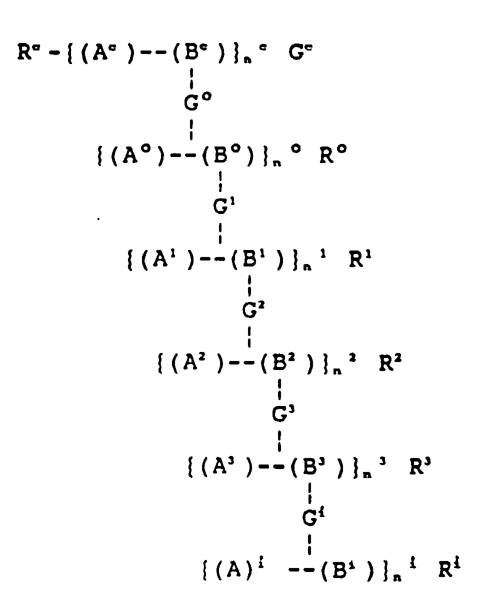 wherein R^{c} is a non-reactive end group and each R^{o}, R¹, R², R³, and R¹ is selected from initiators selected from a group consisting of free radical initiators, cationic initiators, anionic initiators, and group transfer initiators; (i) represents repetitive linear polymers having the unit formula {(Aⁱ)--(Bⁱ)} ; A^{c}, A^{o}, A¹, A², A³, and Aⁱ are non-reactive comonomers or, oligomers or polymers formed from a polymerizable monomer, said oligomers or polymers being capable of withstanding the conditions required for preparation of a graft polymer; B^{c}, B^{o}, B¹, B², B³, and Bⁱ are protected or unprotected reactive nucleophilic or electrophilic monomers or, oligomers or polymers formed from a polymerizable monomer, said oligomers or polymers being capable of withstanding the conditions required for preparation of a graft polymer; G is a terminating group or a grafting component; n^{c} is the degree of polymerization of a core initiator; n^{o} is the degree of polymerization of a first comb branch; n¹ is the degree of polymerization of a first generation comb-burst branch; n² is the degree of polymerization of a second generation comb-burst branch; n³ is the degree of polymerization of a third generation comb-burst branch, nⁱ is the degree of polymerization of the i^{th} generation comb-burst polymer having at least one branch point; wherein nⁱ ≥ 2 for the case where i = c, ^{o}, and 1, and nⁱ ≥ 2 if nⁱ⁺¹ is > zero, the largest i for which nⁱ does not equal zero is the total generation level of the polymer wherein the superscripts c, ^{o}, 1, 2, 3, and i designate comb-burst generation level; the unit ratio of A units to B units in any {(A)--(B)} segment of the polymer is 0 to 1:100 to 1.

As indicated above, each of R^{o}, R¹, R², R³, and Rⁱ in these inventive polymers is selected as a moiety from a radical initiator, a moiety from a cationic initiator, a moiety from an anionic initiator, or a group transfer initiator. R^{o}- Rⁱ can be for example hydrogen, an alkyl group, Lewis acids, or the like.

The Gⁱ group is the grafting component formed by the reaction of the living end, or a derivative of the living end, of the i^{th} generation oligomer with the reactive groups of the (i-1) generation material. Thus, an anionic oligomer may be reacted directly with an electrophilic precursor generation, or it may be terminated by, for example, a halogen such as chlorine, bromine, or iodine, to create an electrophilic end group for grafting to a nucleophilic precursor. Similarly, a cationic oligomer may be reacted directly with a nucleophilic precursor generation, or terminated with, for example, water, hydrogen sulfide, or an amine to give a nucleophilic end group for reaction with an electrophilic precursor. In the case of G^{c}, the "graft" is to a monofunctional molecule, which may be as simple as quenching the active end with a proton or hydroxide, as would be the case with normal termination of ionic oligomers with water, or trapping with a specific molecule in order to introduce a single desired functional group to the molecule. Other telechelic groups suitable for grafting purposes may be found in Goethals, "Telechelic Polymers"; Syn. Appln., CRC Press (1989).

The oligomeric and polymeric segments of these materials can be homopolymers or copolymers, it being understood that the formulae herein represent bonding of the grafting G groups to either segment A, if it is present, or to segment B, and it being further understood that the grafting to any A segment is at the terminal end of the molecule, any other segment A grafting would result in the potential for crosslinking the polymers, which is not part of the invention herein. Also, for purposes of this invention, each A segment can be monomeric or, oligomers or polymers formed from polymerizable monomers, the only condition being that the said monomers, oligomers and polymers must be capable of withstanding the conditions required for preparation of subsequent graft junctures. As illustrated in the formulae, the bond from G to the next generation is indicated by a vertical line about halfway between the A segments and the B segments to illustrate that G can be bonded to either A, if it is present, or to B, which is always present in the molecule.

Segments of A include for example, -CH₂CH₂-, -CH₂ CH=CHCH₂-, -CH₂C(CH₃)₂-, -CH₂CH(CN)-, 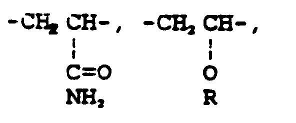 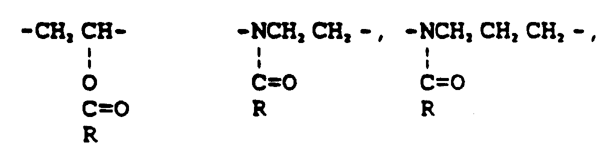 -OCH₂CH₂-, -SCH₂CH₂-, -R'₂SiO-, 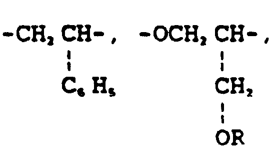 wherein R' is an alkyl group of 1 to 4 carbon atoms, aryls, arylalkyl, hydrogen, or carboalkoxy and R is an alkyl group of 1 to 4 carbon atoms, aryls, or hydrogen; 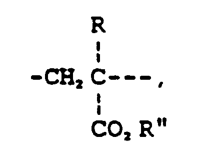 wherein R has the same meaning as set forth above, and wherein R'' can be an alkyl group of 1 to 4 carbon atoms.

Preferred as A segments are -CH₂CH₂-, 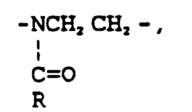 -CH₂C(CH₃)₂-, 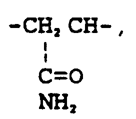 -CH₂CH₂O-, -CH₂CH₂S-, -CH₂CH=CHCH₂-, -R'₂SiO-, 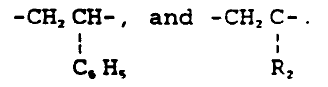 Most preferred are the A segments

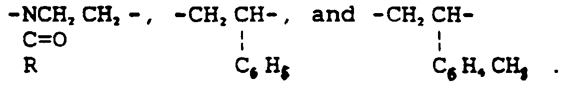

Each B segment can be monomeric, or oligomers or polymers formed from polymerizable monomers, wherein said monomers, oligomers and polymers must be capable of withstanding the conditions required for preparation of a graft polymer and further, the B segments must contain at least one unit which is nucleophilic or electrophilic in character.

The groups B contain the reactive sites to which the oligomers may be grafted. In many cases, these groups may need to be present in latent or masked form if they would otherwise be incompatible with the oligomerization process. For example, polymerization of ethyleneimine leads to highly branched polyethyleneimine oligomers which are not useful for this invention because the secondary amines formed are also reactive under the polymerization conditions. Oxazoline polymerization leads to linear polyethyleneimine in a protected form, and the secondary amines can be unmasked for grafting by hydrolysis. For alkylene oxide oligomerizations, hydroxyl groups intended for use as future graft sites would need to be masked as, for example, an ether to preclude the possibility of forming highly cross-linked gel systems. An example of a latent reactive site would be an alcohol group of a polyol which would require activation by conversion to a halide or sulfonate to allow reaction with anionic oligomer.

Thus, B as a nucleophile can be selected from such groups as 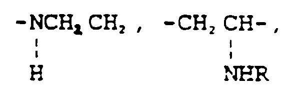 -CH₂CH(OH)-, -CH₂CH(SH)-, -OCH₂CH(CH₂OH)-, and 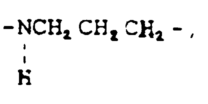 while B as an electrophile can be selected from such groups as 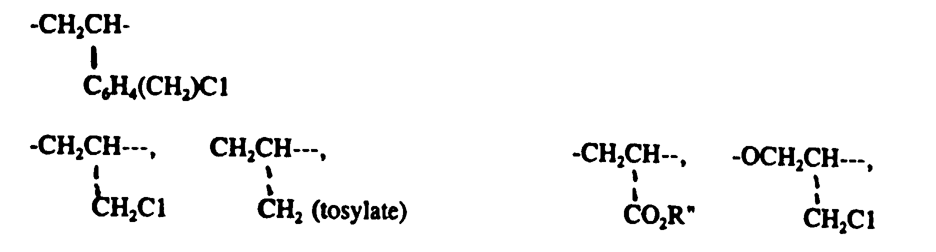 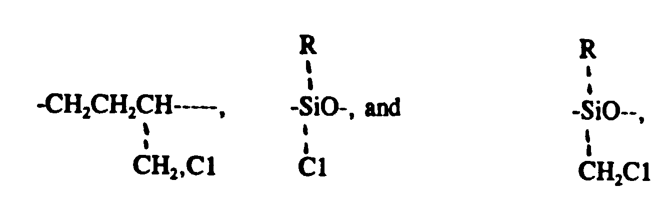 wherein R and R" have the meanings set forth above.

It should be understood that homopolymers consist of only the B segment, while copolymers can be had by combining the B segments with the A segments. Copolymers can also be prepared by using different monomers for the B segment of different generations, for example B¹ being different from B².

The inventors herein contemplate that for purposes of this invention, there must be at least one B segment and therefore the ratio of A segments to B segments ranges from 0 to 1 to 100 to 1.

This invention also comprises a process for preparing non-crosslinked poly-branched polymers having the general formula 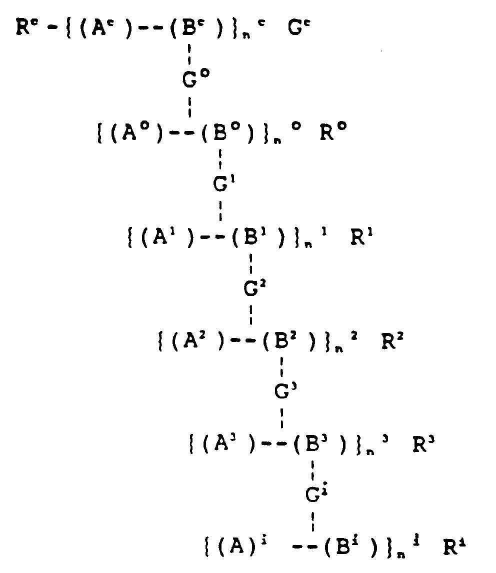 wherein R^{c} is a non-reactive end group and wherein each R^{o}, R¹, R², R³, and Rⁱ is selected from initiator types selected from a group consisting of free radical initiators, cationic initiators, anionic initiators, and group transfer initiators; (i) represents repetitive linear polymers having the unit formula {(Aⁱ)--(Bⁱ)} ; A^{c}, A^{o}, A¹, A², A³, and Aⁱ are non-reactive comonomers or, oligomers or polymers formed from a polymerizable monomer, said oligomers or polymers being capable of withstanding the conditions required for preparation of a graft polymer; B^{c}, B^{o}, B¹, B², B³, and Bⁱ are protected or unprotected reactive nucleophilic or electrophilic monomers or, oligomers or polymers formed from a polymerizable monomer, said oligomers or polymers being capable of withstanding the conditions required for preparation of a graft polymer; G is a terminating group or a grafting component; n^{c} is the degree of polymerization of a core initiator; n^{o} is the degree of polymerization of a first comb branch; n¹ is the degree of polymerization of a first generation comb-burst branch; n² is the degree of polymerization of a second generation comb-burst branch; n³ is the degree of polymerization of a third generation comb-burst branch, nⁱ is the degree of polymerization of the i^{th} generation comb-burst polymer having at least one branch point; wherein nⁱ ≥ 2 for the case where i = c, ^{o}, and 1, and nⁱ ≥ 2 if nⁱ⁺¹ is > zero, the largest i for which nⁱ does not equal zero is the total generation level of the polymer wherein the superscripts c, ^{o}, 1, 2, 3, and i designate comb-burst generation level; the unit ratio of A units to B units in any {(A)--(B)} segment of the polymer is 0 to 1:100 to 1, the process comprising (I) forming a linear initiator core having at least one reactive site and having the general formula
R^{c}-{(A^{c})--(B^{c})}n^{c} G^{c} ; (II) reacting all or part of the sites (B^{c}) ) of (I) with a reactive polymer having the unit formula G^{o}{(A^{o})--(B^{o})}n^{o}R^{o} to form multiple branches that contain at least one reactive site on each branch using protection-deprotection reactions to ensure that the unit formula G^{o}{(A^{o})--(B^{o})}n^{o}R^{o} reacts only with (B^{c}) sites of (I) and that no reactions occur at the reactive sites B^{o}; (III) repeat (II) sequentially to form successive generations of reactive branches to give the desired non-crosslinked poly-branched polymers.

It should be noted by those skilled in the art that the polymer requires an initiator core (initiator core molecule). This initiator core may or may not be a "living polymer" or "living oligomer", which oligomers and/or polymers are generally known to those skilled in the art. "Living systems" are preferred in order to control polydispersity of the comb-burst dendrimers. Using specific chemistry, the inventors herein can explain this aspect of the invention beginning with reference to "Polymeric Amines And Ammonium Salts", edited by E.J. Goethals, Pergamon Press, (1980), with especial reference to pages 55 et seq. wherein there is taught one method of producing living polymers in a paper entitled "Linear Polyalkylenimines", Saegusa, T. and Kobayashi, S.

Using the example of Saegusa, page 58, one can observe that an initiator such as methyl iodide is first reacted with an oxazoline in the following sequence to give an oligomeric "living oligomer" having, in this case, two protected reactive sites designated as 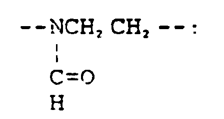 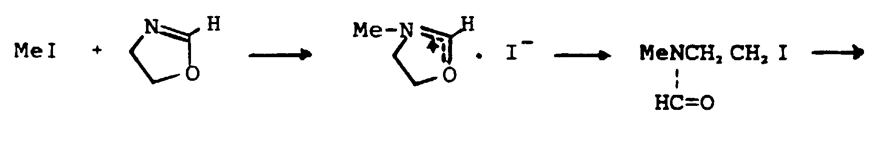 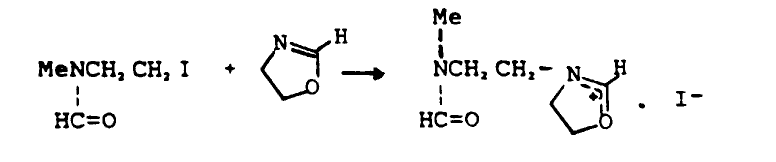 that is,

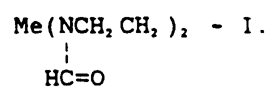

With further reference to Fig. 1 of the instant invention, the initiator core in the specific case described just above would be shown in Fig. 1 as R^{c}(B^{c})ₙc G^{c}; where R^{c} is methyl and G^{c} is as described above.

Reaction sequences are then chosen to deprotect the nitrogen groups so that each of the two reactive sites adds a reactant possessing its own, new reactive site, or sites, which introduces multiplicity, to obtain a "dendrimer" -{(A^{o})--(B^{o})}n^{o}-R^{o} of generation 0 (see Fig. 1), wherein "dendrimer" has the same or similar meaning as that used by Tomalia, et. al. in the article referenced supra. As can be observed from the reaction sequence set forth above, this process requires that protection-deprotection strategies are used to ensure that the reactant reacts with all reactive (B^{c}) sites, but does not react any (B^{o}) sites. Protection-deprotection strategies are generally known to those skilled in the art and great detail does not have to be set forth here. Suffice it to suggest that the living oligomer set forth above has the protective group 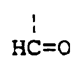 on each nitrogen of the oligomer whereupon the oligomer is then hydrolyzed with an acid to give polymeric units having reactive amine groups i.e. 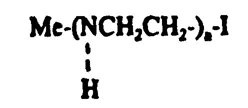 which are then used as the reactive sites to form the next generation, it being understood that the reactive sites of the polymer being grafted to the amine groups are protected before this reaction takes place, and that they too are hydrolyzed after the grafting reaction to give additional reactive sites for the next generation of branching. Additional iterative sequences involving addition of new reactants having reactive sites is then undertaken in order to add branches onto branches to form the poly-branched polymer of this invention until the polymers will not form due to steric hinderence referred to as comb-burst dense packing. The article by Tomalia, et al, referenced supra sets forth such technical terms.

One of the inventive processes used to prepare polymers of this invention relies on the polymerization of oxazoline based polymers, including for example poly-2-aryl substituted oxazolines, poly-2-alkyl substituted oxazolines, the latter including specifically 2-ethyl-2-oxazoline. Methyl p-toluenesulfonate has been shown to polymerize oxazolines and the polymerization mechanism has been determined to be cationic, producing a "living polymer". This allows the preparation of polymer samples with well defined molecular weight and low polydispersity. The end of the growing polymer chain contains an oxazolinium ion as disclosed above, that can be trapped by a variety of nucleophiles. To graft the living poly(2-ethyl-2-oxazoline) chains, they are terminated with the secondary amine groups contained on linear poly(ethyleneimine)(LPEI). After grafting onto the linear poly(ethyleneimine) has been accomplished, hydrolysis of the poly(2-ethyl-2-oxazoline) grafts will generate poly(ethyleneimine) branches. This allows further living poly(2-ethyl-2-oxazoline) chains to be grafted onto the poly(ethyleneimine) branches. Repetition of the grafting and hydrolysis forms the inventive polymers with the structures shown herein.

### Example 1

A 250 ml one-necked round-bottomed flask equipped with a magnetic stirring bar and a Dean-Stark trap that was surmounted with a reflux condenser was charged with 2.84 gm (15.3 mmole) of methyl tosylate and 125 ml of toluene. The mixture was heated at reflux and solvent was collected until all water had been removed. At this time, 30.0 gm (303 mmoles) of freshly distilled 2-ethyl-2-oxazoline was added all at once and the mixture was refluxed for approximately 4 hours. During this time, in a separate flask, 1.64 gm (38.1 mmole of repeat units) of linear poly(ethyleneimine) was azeotropically dried with toluene. When the poly(ethyleneimine) was dry it was added to the round-bottomed flask containing the oxazoline oligomer and then allowed to reflux for an additional 3 hours. Any ungrafted living poly(2-ethyl-2-oxazoline) chains were neutralized by the addition of 2.0 ml of water with refluxing for an additional 1 hour. Toluene was removed under reduced pressure to leave a yellowish oily solid that was dissolved in chloroform and precipitated dropwise into diethyl ether. The yellow solid was filtered from solution and dried overnight in a vacuum oven to yield 29.7 gm (94% yield) of grafted poly(2-ethyl-2-oxazoline) as a yellow powder.

### Example 2

Into a 500 ml one-necked round-bottomed flask was placed 21.6 gm of the oxazoline from example 1 and 350 ml of water. When the polymer had completely dissolved, 35 ml of concentrated sulfuric acid was added. The flask was equipped with a distillation head and the mixture was heated at reflux and distillate was collected until propionic acid could not be detected. Water was added to the distilling pot when the volume was reduced to less than approximately 75 ml. Upon removal of the propionic acid the distillation head was replaced with a reflux condenser surmounted with a pressure equalized addition funnel charged with 5N NaOH. The base was slowly dripped into the reaction mixture maintained at reflux. When the pH of the reaction mixture was approximately 12, heating was discontinued. At room temperature a solid had formed at the surface of the aqueous mixture. This precipitate was removed and placed in a 250 ml round-bottomed flask with 175 ml of toluene. The water was removed from the water-toluene azeotrope by distillation. When water removal was complete, the solid became soluble in the refluxing toluene. The hot toluene solution was poured into a 250 ml round-bottomed flask leaving behind insoluble salts. Toluene was removed under reduced pressure to leave a brownish, waxy solid. The sample was dried for approximately 24 hours under vacuum to give 9.14 gm (97% yield) of polymer sample.

### Example 3

Using the general method of Example 2, hydrolysis of the graft polymers, was carried out on a separate batch of the graft polymers in the following manner. Five grams (5.0 gm) of the graft copolymer were placed in a 250 ml round-bottomed flask with 100 ml of water and 10 gm of sulfuric acid. The flask was heated with a heating mantle to give a slow distillation of the propionic acid/water azeotrope. The distillation was continued for 2 days, with water being added as necessary to maintain the reaction volume. Approximately 200 ml of distillate was collected over the course of the hydrolysis. The heating was discontinued and 50% NaOH was added slowly to bring the pH to 10. The free polyamine was insoluble in the saturated salt solution, giving a separate phase on top of the aqueous solution. The phases were separated and the polyamine was placed in a 250 ml round-bottomed flask. One hundred fifty ml of toluene was added and a Dean-Stark trap was attached. After reflux overnight (about 16 hours), no more water was being removed and the polyamine had dissolved in the hot toluene. The hot solution was filtered and the solvent was removed from the filtrate using vacuum and agitation to give branched poly(ethyleneimine) weighing 2.2 gm (100% of theory) as an orange oil. The ¹³C-NMR spectrum showed a peak for linear poly(ethyleneimine) (49.4 ppm/intensity 8075), residual unhydrolyzed propionamide (9.5 ppm/intensity 156),(26.3 ppm/intensity 180), and primary amine end group (41.7 ppm/intensity 61). No peak for a hydroxy terminal group was observed. While the intensities may not be interpreted as a quantitative measure of the groups present, qualitatively, hydrolysis was 80 to 90% complete and grafting was complete within the limits of detection.

### Example 4

A 2 liter, 3-necked, round-bottomed, glass flask was used with a shaft driven stirrer, instead of magnetic stirring. The initial loading was: water - 250 ml, material prepared essentially by the method of example 3 - 125 gm, sulfuric acid - 150 gm. Additional sulfuric acid, 100 gm was added halfway through the hydrolysis to improve solubility. Internal flask temperature was monitored and a solenoid valve was rigged to add water whenever the temperature rose above 107°C. Thus, constant attention was not necessary and the distillation could be left unattended overnight. The heating mantle was also set to shut off at the same temperature so that the flask would not overheat if the water reservoir ran out of water. After 2 days of continuous distillation, 1.6 liters of distillate was collected. The reaction mixture was neutralized and the polymer phase was separated. The crude polymer was purified by dissolving in hot water (1 liter) and precipitated by slow addition to cold water. After two precipitations, the supernatant solution was neutral to Hydrion^{R} paper. The resulting hydrated polymer was dehydrated via toluene azeotrope as described above to give LPEI (51 gm 94% yield). The ¹³C-NMR spectrum showed LPEI with residual amide carbon intensities 0.5% of the LPEI intensity. Primary amine end group intensity was 0.4% of the LPEI intensity.

### Example 5

Into a 250 ml round-bottomed glass flask was placed p-toluenesulfonic acid monohydrate (2.0 gm, 11 mmole) and toluene (100 ml). A Dean-Stark trap was attached and the mixture was heated at reflux until water removal was complete. Ethyl oxazoline (10 gm, 100 mmole) was added all at once and the reflux was continued for 2 hours. LPEI (1.0 gm, 23 meq.) was placed in toluene (25 ml) and the mixture was heated to boiling to dissolve the polymer and azeotropically remove trace water in the polymer. The hot LPEI solution was added all at once, to the cloudy oligomer suspension. An orange oil began to precipitate immediately. After 1 hour at reflux, the mixture was cooled and the solvent stripped using vacuum. The residue was dissolved in CH₂Cl₂ (40 ml) and precipitated by a slow addition to ether (500 ml). The solid was collected by filtration and dried in a vacuum oven at 40° to 50°C to give the grafted polymer (12 gm, 92% yield) as a yellow powder. At higher M/I ratios, the oligomerization time had to be increased to allow complete coversion of the ETOX. For example, intermediate degree of polymerization runs (M/I = 200, olig. time = 3 hours. or M/I = 400, olig. time = 6 hours) had low yields due to incomplete conversion. Increasing the reaction time to 12 hours and 24 hours respectively, gave higher conversions and yields. The highest M/I (1000) run, had an oligomerization time of 36 hours, which was not long enough for complete conversion. This gave a material with actual oligomer dp of 700. The ¹³C-NMR spectrum of the poly-branched polymer derived from this material showed a peak for primary amine end groups which was approaching the limits of detection for the signal/noise ratio. No hydroxyl terminal group was detectable.

## Claims

1. A composition of matter comprising non-crosslinked poly-branched polymers having the general formula 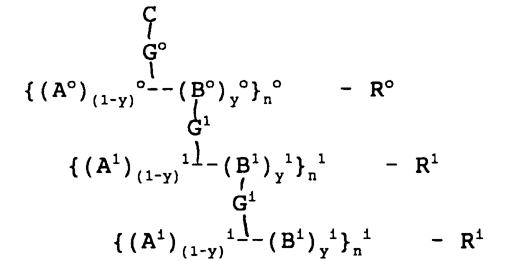 wherein:
C is a core molecule;
each R is the residual moiety of an initiator selected from a group consisting of free radical initiators, cationic initiators, anionic initiators, Lewis acid and group transfer initiators;
A and B are polymerizable monomers or comonomers capable of withstanding the conditions required for preparation of a graft polymer or for preparation of subsequent graft junctures;
each G is a grafting component, and the designation 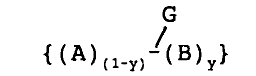 indicates that G can attach to either an (A) unit or a (B) unit;
n is the degree of polymerization of the indicated generation comb branches,
y is the fraction of B units in the indicated generation branch, and has a value of .01 to 1;
the superscripts 0, 1 and i designate the comb branch generation level;
and at least n^{o} and n¹ are > 2.

2. A composition of matter as claimed in claim 1 wherein the B segment has the unit formula 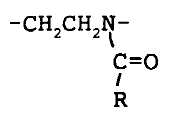 during polymerization of the B segment wherein R is an alkyl group of 1 to 4 carbon atoms, aryl or hydrogen.

3. A composition of matter as claimed in claim 2 which is a hydrolysis product.

4. A composition of matter as claimed in claim 1 wherein the A segment has the unit formula 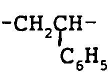 and wherein the B segment has the unit formula

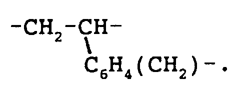

5. The composition of claim 1 in which said polymerizable monomers or comonomers in a given generation of branches are capable of withstanding the conditions required for grafting thereto by being protected from such grafting at least during said {A--B} polymerization;
at least said B monomers or comonomers having been deprotected subsequent to polymerization and grafting to said core or a prior branch.

6. The composition of claim 5 in which said B monomers or comonomers in a given branch are protected during said polymerization, and are subsequently deprotected by hydrolysis.

7. The composition of claim 5 in which said B monomers or comonomers in a given branch are protected during said polymerization and are deprotected by adding a branch or graft reactive group thereto.

8. A composition of matter as claimed in claim 1 in which:
A is (-CH₂CH₂NH-)ₓ and B is 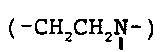 and wherein each of said branches is formed from monomers which leave a protective member on each nitrogen atom in the branch whereby grafting to said branch or during its grafting to a prior branch or to the core, at least some of which are subsequently removed from said branch by a deprotection step performed subsequent to polymerization and grafting of said branch to a prior branch or to said core.

9. A composition of matter as claimed in claim 8 in which said deprotection is effected by hydrolysis.

10. A composition of matter as claimed in claim 8 wherein said protective member on said nitrogen atom is 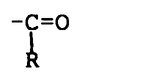 wherein R is an alkyl group of 1 to 4 carbon atoms, aryl or hydrogen.

11. A composition as claimed in claim 8 wherein linear polyethyleneimine is reacted with oxazoline oligomers and is grafted thereby.

12. A composition as claimed in claim 11 wherein the oxazoline oligomer is poly-2-alkyl substituted oxazoline.

13. A composition as claimed in claim 11 wherein the oxazoline oligomer is poly-2-aryl substituted oxazoline.

14. A composition as claimed in claim 13 wherein the grafted polymer is hydrolyzed after grafting.

15. A composition as claimed in claim 12 wherein the grafted polymer is hydrolyzed after grafting.

16. A composition as claimed in claim 11 wherein the grafted polymer is hydrolyzed after grafting.

17. A process for producing non-crosslinked poly-branched polymers having the general formula 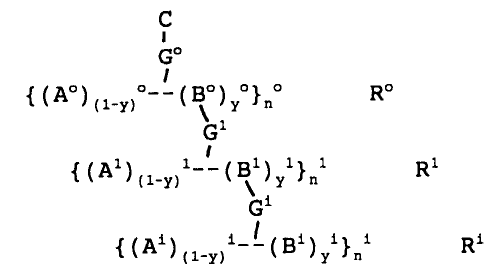 wherein:
C is a core molecule;
each R is the residual moiety of an initiator selected from a group consisting of free radical initiators, cationic initiators, anionic initiators, Lewis acid and group transfer initiators;
A and B are polymerizable monomers or comonomers capable of withstanding the conditions required for preparation of a graft polymer or for preparation of subsequent graft junctures;
each G is a grafting component, and the designation 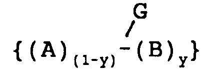
indicates that G can attach to either an (A) unit or a (B) unit;
n is the degree of polymerization of the indicated generation comb branches,
y is the fraction of B units in the indicated generation branch, and has a value of .01 to 1;
the superscripts 0, 1 and i designate the comb branch generation level;
and at least n^{o} and n¹ are > 2;
said process comprising
(I) forming a core having at least one reactive site;
(II) reacting essentially all of the reactive sites of said core with a reactive polymer having the unit formula 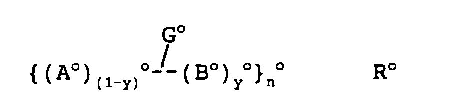 to form multiple branches which contain reactive (B^{o}) sites on each branch, using a reactive scheme such that the reactive monomer units (B^{o}) are capable of withstanding the conditions required for preparation of a graft polymer to ensure that said reactive polymer 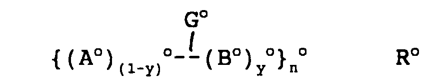 reacts with said reactive sites of said core, but that no reactions occur at said (B^{o}) sites;
(III) repeating step (II) sequentially by reacting reactive polymer having the unit formula 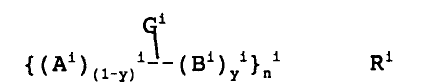 with the reactive sites of said polymerizable B⁽ⁱ⁻¹⁾ monomers or comonomers of the previous generation to form successive generation of branches to give the desired non-crosslinked poly-branched polymer.

18. The process of claim 17 in which linear poly lene amine is used as said initiator core and oxazoline based oligomers are grafted thereto to form zero generation branches having nonreactive oxazoline groups thereon, said generation branches then being hydrolyzed to form reactive amine sites to which first generation oxazoline oligomers are then grafted, said hydrolysis and subsequent reaction steps being then sequentially reiterated to form the desired crosslinked poly-branched polymer.

19. A process as claimed in claim 18 wherein the oxazoline oligomer is poly-2-alkyl substituted oxazoline

20. A process as claimed in claim 18 wherein the oxazoline oligomer is poly-2-aryl substituted oxazoline.

## Patentansprüche

1. Material-Zusammensetzung, das nicht-vernetzte, mehrfach verzweigte Polymere der folgenden allgemeinen Formel umfaßt 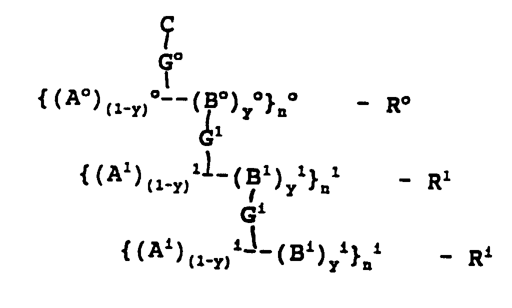 worin
C ein Kern-Molekül darstellt,
R jeweils die restliche Gruppe eines Starters ist, der ausgewählt ist aus einer Gruppe bestehend aus freien Radikalstartern, kationischen Startern, anionischen Startern, Lewis-Säure- und Gruppentransfer-Startern,
A und B polymerisierbare Monomere oder Comonomere sind, die den zur Herstellung eines Pfropfpolymers oder zur Herstellung von nachfolgenden Pfropfverbindungen erforderlichen Bedindungen widerstehen können,
G jeweils eine Pfropfkomponente ist, wobei die Bezeichnung 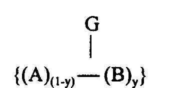 zeigt, daß G an eine (A)-Einheit oder eine (B)-Einheit gebunden sein kann,
n das Maß der Polymerisation der angegebenen Kamm-Verzweigungs-Generation ist,
y der Teil an B-Einheiten in der angegebenen Kamm-Verzweigungs-Generation ist und einen Wert von 0,01 bis 1 besitzt,
wobei die hochgestellten Werte 0, 1 und i den Rang der Kamm-Verzweigungs-Generation bezeichnen,
und wobei mindestens n^{o} und n¹ > 2 sind.

2. Materialzusammensetzung nach Anspruch 1, bei der das B-Segment während der Polymerisation des B-Segments die Einheitsformel -CH₂CH₂N(COR)- aufweist, worin R eine Alkylgruppe mit 1 bis 4 Kohlenstoffatomen, Aryl oder Wasserstoff ist.

3. Materialzusammensetzung nach Anspruch 2, welche ein Hydrolyseprodukt ist.

4. Materialzusammensetzung nach Anspruch 1, bei der das A-Segment die Einheitsformel -CH₂C(C₆H₅)H- aufweist und worin das B-Segment die Einheitsformel -CH₂-C(C₆H₄(CH₂)- aufweist.

5. Zusammensetzung nach Anspruch 1, worin die polymerisierbaren Monomere oder Comonomere in einer bestimmten Verzweigungsgeneration den zum Pfropfen daran erforderlichen Bindungen widerstehen können, indem sie gegenüber einem derartigen Pfropfen mindestens während der {A--B}-Polymerisation geschützt sind, wobei mindestens bei den B-Monomeren oder -Comonomeren die Schutzgruppen nach der Polymerisation oder nach dem Pfropfen an den Kern oder an eine bestehende Verzweigung entfernt werden.

6. Zusammensetzung nach Anspruch 5, bei der die B-Monomere oder -Comonomere in einer bestimmten Verzweigung während der Polymerisation geschützt sind und die Schutzgruppen anschließend durch Hydrolyse entfernt werden.

7. Zusammensetzung nach Anspruch 5, bei der die B-Monomere oder Comonomere in einer bestimmten Verzweigung während der Polymerisation geschützt sind und die Schutzgruppen durch Anfügen einer Verzweigung oder einer reaktiven Pfropfgruppe daran entfernt werden.

8. Materialzusammensetzung nach Anspruch 1, worin:
A (-CH₂CH₂NH-)ₓ und B (-CH₂CH₂N-)- ist, und worin jede Verzweigung aus Monomeren gebildet wird, die an jedem Stickstoffatom in der Verzweigung während des Pfropfens an die Verzweigung oder während ihres Pfropfens an die vorhergehende Verzweigung oder an den Kern eine Schutzgruppe zurücklassen, von denen mindestens einige nachfolgend durch einen Schritt der Entfernung der Schutzgruppe, der nach der Polymerisation und dem Pfropfen der Verzweigung an eine vorhergehende Verzweigung oder an den Kern durchgeführt wird, von der Verzweigung entfernt werden.

9. Materialzusammensetzung nach Anspruch 8, bei der die Entfernung der Schutzgruppe durch Hydrolyse bewirkt wird.

10. Materialzusammensetzung nach Anspruch 8, bei der die Schutzgruppe am Stickstoffatom -CO-R ist, wobei R eine Alkylgruppe mit 1 bis 4 Kohlenstoffatomen, Aryl oder Wasserstoff ist.

11. Zusammensetzung nach Anspruch 8, bei der lineares Polyethylenimin mit Oxazolinoligomeren umgesetzt und dadurch gepfropft wird.

12. Zusammensetzung nach Anspruch 11, bei der das Oxazolinoligomer Poly-2-alkyl-substituiertes Oxazolin ist.

13. Zusammensetzung nach Anspruch 11, bei der das Oxazolinoligomer Poly-2-aryl-substituiertes Oxazolin ist.

14. Zusammensetzung nach Anspruch 13, bei der das Oxazolinoligomer nach dem Pfropfen hydrolysiert wird.

15. Zusammensetzung nach Anspruch 12, bei der das gepfropfte Polymer nach dem Pfropfen hydrolysiert wird.

16. Zusammensetzung nach Anspruch 11, bei der das gepfropfte Polymer nach dem Pfropfen hydrolysiert wird.

17. Verfahren zur Herstellung nicht-vernetzter, mehrfach verweigter Polymere mit der folgenden allgemeinen Formel: 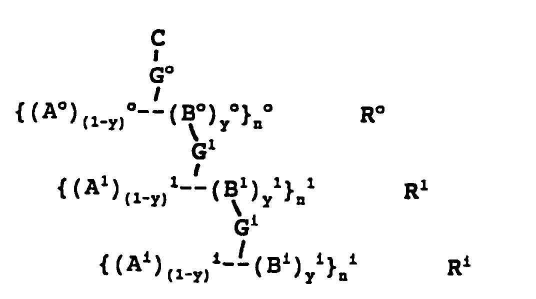 worin:
C ein Kern-Molekül darstellt,
R jeweils die restliche Gruppe eines Starters ist, ausgewählt aus der Gruppe bestehend aus freien Radikalstartern, kationischen Startern, anionischen Startern, Lewis-Säure- und Gruppentransfer-Startern,
A und B polymerisierbare Monomere oder Comonomere sind, die den zur Herstellung eines Pfropfpolymers oder zur Herstellung von nachfolgenden Pfropfverbindungen erforderlichen Bedindungen widerstehen können,
G jeweils eine Pfropfkomponente ist und die Bezeichnung 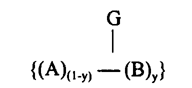 zeigt, daß G an eine (A)-Einheit oder eine (B)-Einheit gebunden sein kann,
n das Maß der Polymerisation der angegebenen Kamm-Verzweigungs-Generation ist,
y der Teil an B-Einheiten in der angegebenen Kamn-Verzweigungs-Generation ist und einen Wert von 0,01 bis 1 besitzt,
die hochgestellten Werte 0, 1 und i den Rang der Kamm-Verzweigungs-Generation bezeichnen,
und wobei mindestens n^{o} und n¹ > 2 sind, wobei das Verfahren umfaßt
(I) Bilden eines Kerns mit mindestens einer reaktiven Stelle,
(II) Umsetzen im wesentlichen aller oder eines Teils der reaktiven Stellen des Kerns mit einem reaktiven Polymer mit der Einheitsformel 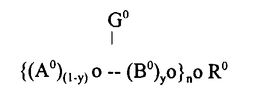 wobei mehrere Verzweigungen gebildet werden, die in jeder Verzweigung reaktive (B⁰)-Stellen enthalten, wobei ein Reaktionsverlauf verwendet wird, so daß die reaktiven (B⁰)-Monomereinheiten den zur Herstellung eines Pfropfpolymers erforderlichen Bindungen widerstehen können, um sicherzustellen, daß das reaktive Polymer 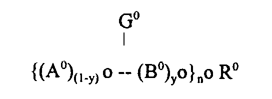 mit den reaktiven Stellen an dem Kern reagieren, wobei keine Reaktionen an den (B⁰)-Stellen ablaufen,
(III) Wiederholen von Schritt (II) nacheinander, durch Umsetzen eines reaktiven Polymers mit der Einheitsformel 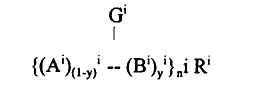 mit den reaktiven Stellen der polymerisierbaren B⁽ⁱ⁻¹⁾-Monomere oder -Comonomere der vorhergehenden Generation, um nachfolgende Verzweigungs-Generationen zu bilden, wobei das gewünschte, nicht-vernetzte, mehrfach verzweigte Polymer erhalten wird.

18. Verfahren nach Anspruch 17, bei dem lineares Polyethylenamin als Starter-Kern eingesetzt wird und auf Oxazolin basierende Oligomere darauf gepfropft werden, um Verzweigungen der Generation 0 mit nicht-reaktiven Oxazolingruppen daran zu bilden, wobei die Verzweigungen der Generation 0 dann hydrolysiert werden, um reaktive Arninstellen zu bilden, an die dann Oxazolin-Oligomere der ersten Generation gepfropft werden, wobei die Hydrolyse und die nachfolgenden Reaktionsschritte nacheinander wiederholt werden, um die gewünschten nichtvernetzten, mehrfach verzweigten Polymere zu bilden.

19. Verfahren nach Anspruch 18, bei dem das Oxazolin-Oligomer Poly-2-alkyl-sustituiertes Oxazolin ist.

20. Verfahren nach Anspruch 18, bei dem das Oxazolin-Oligomer Poly-2-aryl-substituiertes Oxazolin ist.

## Revendications

1. Composition de matière comprenant des polymères multiplement ramifiés non réticulés ayant la formule générale 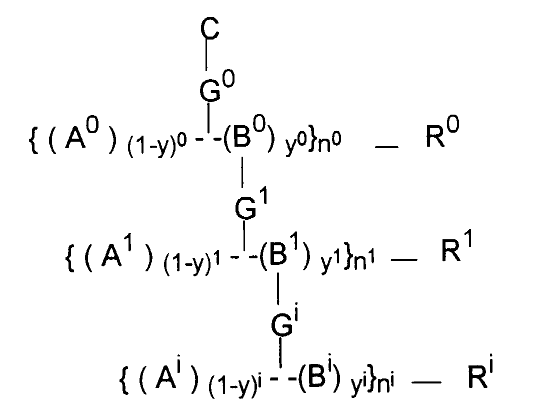 dans laquelle:
C est une molécule noyau;
chaque R est le segment résiduel d'un initiateur choisi parmi un groupe formé par des initiateurs de radicaux libres, des initiateurs cationiques, des initiateurs anioniques, des initiateurs acides de Lewis et de transfert de groupes;
A et B sont des monomères ou comonomères polymérisables capables de résister aux conditions requises pour la préparation d'un polymère greffé ou pour la préparation de jonctions greffées suivantes;
chaque G est un constituant de greffage, et la désignation 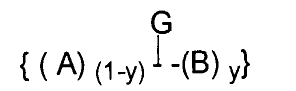 indique que G peut être relié soit à une unité (A) soit à une unité (B); n est le degré de polymérisation des branches de peigne de génération indiquée,
y est la fraction des unités B dans la branche de génération indiquée, et a une valeur de 0,01 à 1;
les exposants 0, 1 et i désignent le niveau de génération de branche de peigne;
et au moins n⁰ et n¹ sont > 2.

2. Composition de matière selon la revendication 1, caractérisée en ce que le segment B pendant la polymérisation du segment B a la formule unitaire 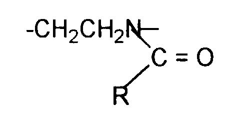 dans laquelle R est un groupe alkyle de 1 à 4 atomes de carbone, aryle ou l'hydrogène.

3. Composition de matière selon la revendication 2, caractérisée en ce qu'il est un produit d'hydrolyse.

4. Composition de matière selon la revendication 1, caractérisée en ce que le segment A a la formule unitaire 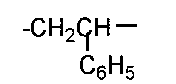 et le segment B a la formule unitaire

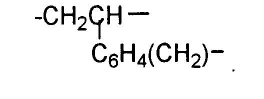

5. Composition selon la revendication 1, caractérisée en ce que lesdits monomères ou comonomères polymérisables dans une génération donnée de branches sont capables de supporter les conditions requises pour le greffage sur ceux-ci en étant protégés d'un tel greffage au moins pendant ladite polymérisation de {A - - B};
au moins lesdits monomères ou comonomères B ayant été déprotégés après la polymérisation et le greffage audit noyau ou une branche antérieure.

6. Composition selon la revendication 5, caractérisée en ce que lesdits monomères ou comonomères B dans une branche donnée sont protégés pendant ladite polymérisation, et sont ensuite déprotégés par hydrolyse.

7. Composition selon la revendication 5, caractérisée en ce que lesdits monomères ou comonomères B dans une branche donnée sont protégés pendant ladite polymérisation et sont déprotégés par addition à ceux-ci d'une branche ou un groupe réactif de ramification.

8. Composition de matière selon la revendication 1, caractérisée en ce que:
A est (-CH₂ CH₂NH-)ₓ et B est 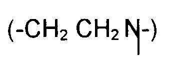 et chacune desdites branches étant formée par des monomères qui laissent un membre protecteur sur chaque atome d'azote dans la branche en se greffant à ladite branche ou pendant son greffage à une branche antérieure ou au noyau, au moins quelques uns d'entre eux étant enlevés ensuite de ladite branche par une étape de déprotection effectuée après la polymérisation et le greffage de ladite branche à une branche antérieure ou audit noyau.

9. Composition de matière selon la revendication 8, caractérisée en ce que ladite déprotection est effectuée par hydrolyse.

10. Composition de matière selon la revendication 8, caractérisée en ce que ledit membre protecteur sur ledit atome d'azote est 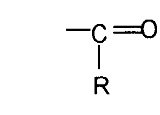 dans lequel R est un groupe alkyle de 1 à 4 atomes de carbone, aryle ou un hydrogène.

11. Composition selon la revendication 8, caractérisée en ce qu'une polyéthylèneimine linéaire est mise à réagir avec des oligomères d'oxazoline et est greffée de cette façon.

12. Composition selon la revendication 11, caractérisée en ce que l'oligomère d'oxazoline est une oxazoline poly-2-alkyle substituée.

13. Composition selon la revendication 11, caractérisée en ce que l'oligomère d'oxazoline est une oxazoline poly-2-aryle substituée.

14. Composition selon la revendication 13, caractérisée en ce que le polymère greffé est hydrolysé après greffage.

15. Composition selon la revendication 12, caractérisée en ce que le polymère greffé est hydrolysé après greffage.

16. Composition selon la revendication 11, caractérisée en ce que le polymère greffé est hydrolysé après greffage.

17. Procédé de préparation de polymères multiplement ramifiés non réticulés ayant la formule générale 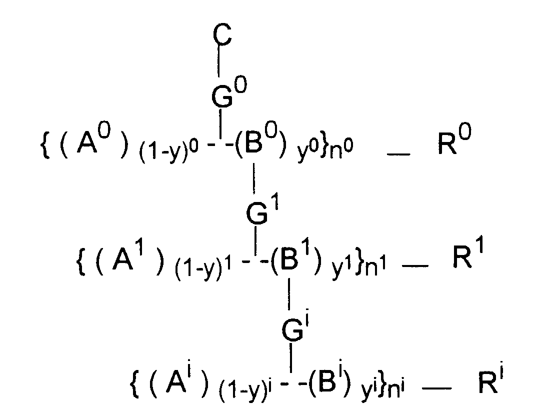 dans laquelle:
C est une molécule noyau;
chaque R est le segment résiduel d'un initiateur choisi parmi un groupe formé par des initiateurs de radicaux libres, des initiateurs cationiques, des initiateurs anioniques, des initiateurs acides de Lewis et de transfert de groupes;
A et B sont des monomères ou comonomères polymérisables capables de résister aux conditions requises pour la préparation d'un polymère greffé ou pour la préparation de jonctions greffées suivantes;
chaque G est un constituant de greffage, et la désignation 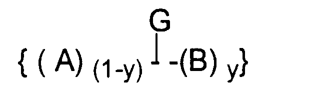 indique que G peut être relié soit à une unité (A) soit à une unité (B);
n est le degré de polymérisation des branches de peigne de génération indiquée,
y est la fraction des unités B dans la branche de génération indiquée, et a une valeur de 0,01 à 1;
les exposants 0, 1 et i désignent le niveau de génération de branche de peigne;
et au moins n⁰ et n¹ sont > 2;
ledit procédé comprenant
(I) la formation d'un noyau ayant au moins un site réactif;
(Il) la réaction de sensiblement tous les sites réactifs dudit noyau avec un polymère réactif ayant la formule unitaire 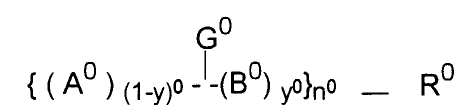 pour former des branches multiples qui contiennent des sites (B⁰) réactifs sur chaque branche, en utilisant un schéma réactionnel tel que les unités monomères réactives (B⁰) soient capables de supporter les conditions requises pour la préparation d'un polymère greffé pour assurer que ledit polymère réactif 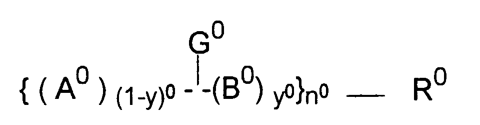 réagisse avec lesdits sites réactifs dudit noyau, mais qu'il n'y ait pas de réactions se produisant sur lesdits sites (B⁰);
(III) la répétition de l'étape (Il) séquentiellement en faisant réagir le polymère réactif ayant la formule unitaire 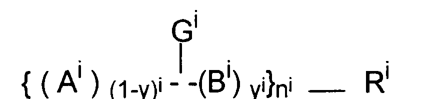 avec les sites réactifs desdits monomères ou comonomères polymérisables (Bⁱ⁻¹) de la génération précédente pour former une génération successive de branches pour donner le polymère multiplement ramifié non réticulé souhaité.

18. Procédé selon la revendication 17, caractérisé en ce qu'on utilise une polyéthylèneamine linéaire comme dit noyau initiateur et que des oligomères à base d'oxazoline sont branchés sur celui-ci pour former les branches de génération zéro ayant des groupes oxazoline non réactifs sur celles-ci, lesdites branches de génération zéro étant ensuite hydrolysées pour former des sites amine réactifs sur lesquels sont ensuite greffés les oligomères oxazoline de la première génération, lesdites étapes d'hydrolyse et de réaction postérieures étant ensuite répétées séquentiellement pour former le polymère multiplement ramifié non réticulé souhaité.

19. Procédé selon la revendication 18, caractérisé en ce que l'oligomère oxazoline est une oxazoline poly-2-alkyle substituée.

20. Procédé selon la revendication 18, caractérisé en ce que l'oligomère oxazoline est une oxazoline poly-2-aryle substituée.
